# DEMANDE DE BREVET EUROPEEN

(11) **EP 1 475 062 A1**
(43) Date de publication de la demande: **10.11.2004**
(21) Numéro de dépôt: 04291101.6
(22) Date de dépôt: 29.04.2004
(51) Int. Cl.: A61F 13/08, A41F 11/16, A41B 11/04

(54) **Article de contention à usage médical équipé d'une bande élastique présentant un revêtement anti-glissement**

(30) Priorité: 07.05.2003 FR 0305549
(71) Demandeur: Cognon-Morin SA, 86100 Chatellerault (FR)
(72) Inventeur: Tendant, Bernard, 25150 Pont-de-Roide (FR)
(74) Mandataire: Jaunez, Xavier

(57) **Abrégé**

L'invention concerne un article de contention à usage médical utilisé pour une jambe ou un bras, comportant une partie principale en tricot élastique servant à comprimer le membre sur lequel l'article est enfilé, laquelle partie principale se prolonge supérieurement par une bande élastique de maintien (20) formée d'une tresse ou d'une dentelle élastique (21) revêtue, du côté intérieur, d'un motif (23) réalisé en un matériau d'anti-glissement.

Conformément à l'invention, le motif d'anti-glissement (23) est constitué par deux bandes rectilignes circonférentielles (24, 25) essentiellement parallèles, agencées à distance l'une de l'autre, et connectées entre elles par une frise alvéolée (26).

## Description

### DOMAINE DE L'INVENTION

La présente invention concerne les articles de contention à usage médical utilisés pour une jambe ou un bras, du type comportant une partie principale en tricot élastique servant à comprimer le membre sur lequel l'article est enfilé pour traiter une insuffisance veineuse ou lymphatique afin d'exercer une pression qui est dégressive du bas vers le haut de l'article.

Plus particulièrement, l'invention s'intéresse aux articles de contention à usage médical du type comportant une partie principale qui se prolonge supérieurement par une bande élastique de maintien formée d'une tresse ou d'une dentelle élastique revêtue, du côté intérieur, d'un motif réalisé en un matériau d'anti-glissement.

### ARRIERE-PLAN DE L'INVENTION

Dans le domaine de la bonneterie, qui n'est pas celui des bas médicaux, on peut citer les documents US-A-3,662,760 et 3,496,944 qui décrivent des moyens d'accrochage du tricot des bas (on ne recherche pas le maintien sur la peau de la personne). Le document US-A-2,514,108 illustre un bas de bonneterie équipée supérieurement d'une bande anti-glisse à motifs caoutchoutés.

Le document FR-A-2.749.167 décrit une orthèse compressive équipée inférieurement d'une bande d'anti-glissement.

Le document DE-U-82 17651.5 décrit un bas médical dont la partie principale se prolonge supérieurement par une bande élastique de maintien. Cette bande de maintien est formée d'un ruban de galon élastique revêtu, du côté intérieur, d'un motif réalisé en un matériau d'anti-glissement, tel que silicone, lequel motif est réalisé sous la forme de points ou de segments agencés de façon discontinue. Il est apparu à l'usage que les motifs de ce type ne confèrent pas une tenue très satisfaisante, en particulier si la jambe n'est pas très ferme ou est revêtue d'une pilosité importante. Il convient de noter subsidiairement qu'un tel ruban présente une structure qui induit un risque de strangulation, certes plus faible qu'avec un bas de bonneterie, mais suffisant pour qu'un bas de ce type soit considéré comme peu performant en bas médical de contention.

Le document FR-A-2 302 054 décrit un bas thérapeutique équipé d'une bande élastique de maintien fixée par surjet à la partie principale du bas. La bande de maintien est constituée par un tissu élastique revêtu intérieurement, pratiquement sur toute sa hauteur, d'un élastomère rainuré. Un tel revêtement ne permet pas d'assurer un anti-glissement efficace, car l'adhérence à la peau est insuffisante avec le serrage autorisé, étant entendu qu'un serrage excessif induirait en effet un risque de strangulation.

Le document US-A-3 800 331 décrit également un bas à bande d'anti-glissement formée par une alternance de zones annulaires tricotées avec un fil élastique ressortant, et de zones de transition à fil vertical. Il est à noter que la bande d'anti-glissement comporte, du côté intérieur, des fils élastiques nus proéminents, ce qui est une source d'inconfort (notamment du fait que les fils pincent les poils).

Plus récemment, la demanderesse a proposé un article de contention à usage médical, dont les fibres constituant la tresse ou la dentelle élastique de la bande de maintien sont choisies de telle façon que ladite bande présente à la fois une caractéristique d'allongement à faible pente, et exerce sur le membre concerné une pression qui est inférieure à celle qu'exerce la partie principale au voisinage de la bande de maintien. Une telle structure est plus performante que celle des bas antérieurs dans la mesure où tout risque de strangulation est écarté, et où la tenue est également assurée de façon relativement satisfaisante par le revêtement, du côté intérieur, avec un motif réalisé en un matériau d'anti-glissement. Cet enseignement, qui est illustré dans le document EP-A-0 621 024, illustre divers motifs d'anti-glissement, tels qu'un ensemble de deux bandes superposées présentant une hauteur d'environ 1,5 cm, ces deux bandes étant séparées par une portion non revêtue de la tresse élastique sur une hauteur d'environ 1 cm. Une telle bande est très satisfaisante pour l'aération dans la mesure où la partie centrale disposée entre les deux bandes de silicone présente une surface très importante. La présence de deux bandes parallèles est également intéressante dans la mesure où elle évite un roulottage des bords, mais il est apparu que la tenue sur le membre s'avère imparfaite, en particulier lorsque le membre qui porte l'article de contention a une structure plutôt ramollie et/ou présente une pilosité importante. Le document présente également d'autres variantes de motifs d'anti-glissement, et l'on peut citer à ce titre une première variante comportant un ensemble de quatre lignes superposées constituées par des successions de points jointifs, la hauteur de chaque ligne individuelle étant d'environ 0,5 cm, ce qui donne une hauteur de motif d'anti-glissement d'environ 2 cm, ou une deuxième variante dans laquelle le motif est constitué de deux lignes sinusoïdales superposées, sans contact l'une avec l'autre, dont la hauteur individuelle est de l'ordre de 0,5 cm, de sorte que le motif d'anti-glissement présente alors une hauteur totale de l'ordre de 1 cm. Là encore, le coefficient d'aération est très satisfaisant dans la mesure où le revêtement en silicone est peu important en surface, mais la tenue en place assurée reste encore imparfaite.

Il convient en outre de noter une difficulté qui est commune à toutes les bandes d'anti-glissement précitées, résidant dans la structure de la dentelle ou tresse élastique utilisée, qui comporte un fil de crin assurant leur tenue mécanique, lequel fil de crin est très dur. Or, le crin peut casser, ou apparaître du côté extérieur ou intérieur par suite d'un déplacement en bordure, provoquant alors une gêne considérable pour le porteur de l'article de contention. Par suite, pour augmenter la longévité du crin et éviter sa sortie du côté extérieur ou intérieur, il convient d'attacher une importance particulière à la surface de broderie. En effet, plus le crin est emprisonné, plus on augmente la stabilisation de celui-ci, de sorte que la broderie doit rester conséquente.

### OBJET DE L'INVENTION

L'invention a pour objet de concevoir un article de contention à usage médical du type précité, dont le motif d'anti-glissement est choisi pour procurer un compromis optimal entre d'une part la tenue assurée par la bande élastique de maintien portant, du côté intérieur, ledit motif réalisé en un matériau d'anti-glissement, et d'autre part le coefficient d'aération de la peau qui est assuré par la surface de la tresse ou dentelle élastique non revêtue.

### DEFINITION GENERALE DE L'INVENTION

Le problème précité est résolu conformément à l'invention grâce à un article de contention à usage médical utilisé pour une jambe ou un bras, comportant une partie principale en tricot élastique servant à comprimer le membre sur lequel l'article est enfilé, laquelle partie principale se prolonge supérieurement par une bande élastique de maintien formée d'une tresse ou d'une dentelle élastique revêtue, du côté intérieur, d'un motif réalisé en un matériau d'anti-glissement, ledit article étant remarquable en ce que le motif d'anti-glissement est constitué par deux bandes rectilignes circonférentielles essentiellement parallèles, agencées à distance l'une de l'autre, et connectées entre elles par une frise alvéolée.

La frise alvéolée permet à la fois d'assurer une résistance satisfaisante au glissement, même lorsque le membre portant l'article de contention est relativement mou ou présente une pilosité importante, et les bandes rectilignes circonférentielles qui encadrent cette frise alvéolée, permettent de garantir la tenue générale de la bande élastique, et en particulier d'éviter tout risque de roulottage au niveau du bord libre de ladite bande élastique.

De préférence, la frise alvéolée a une surface totale qui est notablement supérieure à celle de la partie associée de la tresse ou dentelle élastique non revêtue. En particulier, on pourra prévoir que la surface totale de la frise alvéolée représente 60 à 70 % de la surface délimitée par les deux bandes rectilignes parallèles.

Avantageusement, la frise alvéolée est agencée selon un tracé périodique tangentant chacune des bandes rectilignes parallèles.

De préférence alors, le tracé périodique comporte au moins une bande d'allure sinusoïdale ou en zig-zag, et en particulier deux bandes sinusoïdales ou en zig-zag superposées en opposition de phase, lesdites bandes sinusoïdales étant en contact mutuel par leurs sommets adjacents, les autres sommets étant quant à eux au contact des bandes rectilignes parallèles.

Il sera en outre avantageux de prévoir que les zones de contact entre bandes adjacentes ont une longueur circonférentielle au moins égale à la largeur des deux bandes rectilignes parallèles, et que la ou chaque bande sinusoïdale ou en zig-zag a une largeur sensiblement égale à la largeur des deux bandes rectilignes parallèles.

D'autres caractéristiques et avantages de l'invention apparaîtront plus clairement à la lumière de la description qui va suivre, concernant un mode de réalisation particulier, en référence aux figures du dessin annexé.

### BREVE DESCRIPTION DES DESSINS

Il sera fait référence aux figures du dessin annexé où :
- les figures 1 et 2 illustrent deux articles de contention conformes à l'invention, respectivement sous forme d'un bas de jambe et d'un manchon de bras, avec leur bande élastique de maintien ;
- la figure 3 illustre à plus grande échelle, un agencement du motif d'anti-glissement conforme à l'invention, qui est prévu sur la périphérie interne de la bande élastique de maintien ;
- la figure 4 est une représentation à plat de la face interne de la bande élastique de maintien, montrant une partie de la longueur de ladite bande, avec un motif à frise alvéolée dont la géométrie et le dimensionnement ont été choisis pour optimiser le coefficient d'enduction et le coefficient d'aération.

### DESCRIPTION DETAILLEE D'UN MODE DE REALISATION PREFERE

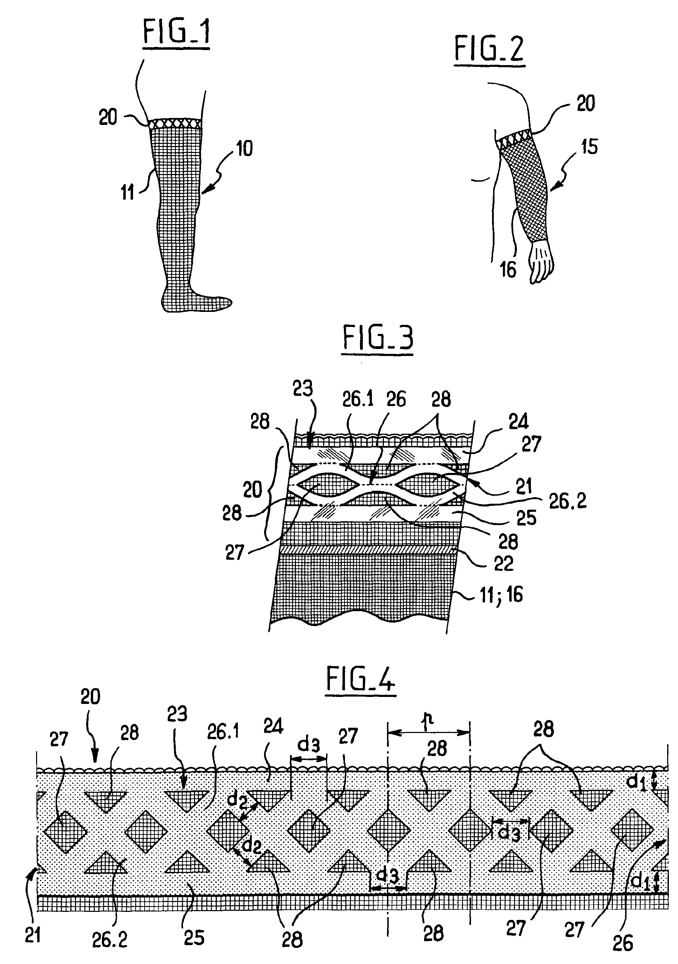

Les figures 1 et 2 illustrent deux articles de contention conformes à l'invention, et l'on distingue ainsi sur la figure 1 un bas de jambe 10 et, sur la figure 2, un manchon de bras 15. Conformément à la pratique habituelle en la matière, chaque article de contention à usage médical 10 ou 15 comporte une partie principale respectivement 11 ou 16 en tricot élastique, servant à comprimer le membre sur lequel l'article est enfilé pour traiter une insuffisance veineuse ou lymphatique, la pression exercée étant dégressive du bas vers le haut de l'article.

De plus, la partie principale 11 ou 16 de l'article de contention à usage médical 10 ou 15 se prolonge supérieurement par une bande élastique de maintien 20, cette bande d'élastique de maintien étant capable d'éviter le glissement de la partie supérieure de l'article de contention 10 ou 20, et ce sans le moindre risque de strangulation du membre concerné qui est comprimé. La bande élastique de maintien 20 est ainsi formée d'une tresse élastique ou d'une dentelle élastique qui est revêtue, du côté intérieur, d'un motif réalisé en un matériau d'anti-glissement, et les fibres constituant la tresse ou la dentelle élastique de cette bande de maintien sont choisies de façon que ladite bande de maintien présente des caractéristiques prédéterminées de souplesse et de pression exercée, conformément à l'enseignement du document EP-A-0 621 024 précité de la demanderesse.

Sur la figure 3, on distingue une partie de la longueur de la bande élastique de maintien 20, comportant en l'espèce une tresse élastique 21 qui est revêtue, du côté intérieur visible sur la figure 3, d'un motif 23 réalisé en un matériau d'anti-glissement, ce matériau étant de préférence constituée par du silicone ou analogue, ici déposé en continu sur la périphérie interne de la bande élastique de maintien 20. La bande élastique de maintien 20, qui est formée d'une tresse ou d'une dentelle élastique 21, est raccordée par une couture 22 à la partie principale 11 ou 16 en tricot élastique de l'article de contention.

Conformément à une caractéristique essentielle de l'invention, le motif d'anti-glissement 23 est constitué par deux bandes rectilignes circonférentielles 24, 25 essentiellement parallèles, agencées à distance l'une de l'autre, et connectées entre elles par une frise alvéolée 26.

La frise alvéolée 26 délimite avec les deux bandes rectilignes circonférentielles 24, 25, des zones non revêtues de silicone, constituant des alvéoles, d'où le nom de frise "alvéolée". On distingue ainsi des alvéoles 27 délimitées entre les parties constitutives de la frise alvéolée 26, et des alvéoles 28 délimitées entre ladite frise alvéolée 26 et l'une ou l'autre des bandes rectilignes circonférentielles 24, 25. Il est alors avantageux de prévoir que la frise alvéolée 26 a une surface totale qui est notablement supérieure à celle de la partie associée de la tresse ou dentelle élastique 21 qui est non revêtue, cette partie associée étant constituée par les différentes alvéoles 27, 28. En particulier, on prévoira que la surface totale de la frise alvéolée 26 représente 60 à 70 % de la surface délimitée par les deux bandes rectilignes parallèles 24, 25.

Comme cela est illustré sur la figure 3, on pourra prévoir que la frise alvéolée 26 est agencée selon un tracé périodique tangentant chacune des bandes rectilignes parallèles 24, 25, et que ce tracé périodique comporte au moins une bande d'allure sinusoïdale ou en zig-zag. En l'espèce, on a représenté un tracé périodique comportant deux bandes sinusoïdales 26.1, 26.2 superposées, en opposition de phase, lesdites bandes sinusoïdales étant en contact mutuel par leurs sommets adjacents, les autres sommets étant quant à eux au contact des bandes rectilignes parallèles 24, 25.

Sur la figure 4, on a représenté plus en détail une frise alvéolée 26 dont l'agencement et le dimensionnement ont été choisis pour conférer un résultat optimal à la fois pour le coefficient d'enduction et le coefficient d'aération.

Les deux bandes 26.1, 26.2 constituant la frise 26 sont alors agencées en zig-zag, et on a noté d2 la largeur de chacune de ces bandes 26.1, 26.2. On a également noté d1 la largeur commune des deux bandes rectilignes parallèles 24, 25, étant entendu que l'on pourra prévoir en variante que ces deux bandes présentent des largeurs différentes. Il est alors intéressant de prévoir que la ou chaque bande sinusoïdale ou en zigzag 26.1, 26.2 a une largeur d2 sensiblement égale à la largeur d1 des deux bandes rectilignes parallèles 24, 25.

On a par ailleurs noté d3 la longueur circonférentielle des zones de contact entre bandes adjacentes, concernant l'une et l'autre des deux bandes 24, 25. Il est alors intéressant de prévoir que les zones de contact entre bandes adjacentes 24, 25, 26.1, 26.2 ont une longueur circonférentielle d3 au moins égale à la largeur d1 des deux bandes rectilignes parallèles 24, 25.

Sur la bande élastique illustrée en figure 4, on a prévu une largeur d1 d'environ 0,7 cm pour chacune des bandes rectilignes parallèles 24, 25, et une largeur d2 de l'ordre de 0,8 cm pour chacune des bandes en zig-zag 26.1, 26.2. Ainsi, si l'on isole une longueur circonférentielle de bande de maintien correspondant au pas p du tracé périodique du motif d'anti-glissement 23, on peut mesurer le rapport entre la surface totale de la frise alvéolée constituée par un tronçon sensiblement en forme de X, et la surface de la tresse ou dentelle élastique 21 non revêtue qui est ici constituée par deux alvéoles 28 et deux demi-alvéoles 27. On arrive ainsi à un rapport d'environ 65 %.

Pour ce qui est des matériaux à utiliser préférentiellement pour réaliser la bande élastique de maintien précitée, on pourra prévoir, pour la tresse ou dentelle élastique, des compositions telles que polyamide-élasthanne-coton, ou polyamide-élasthanne ou encore polyamide-polyester-élasthanne. Le motif choisi sera sélectionné à la fois pour son coefficient maximal d'aération et aussi pour l'esthétique conférée du côté externe de la bande élastique de maintien.

Pour le matériau d'anti-glissement, il sera intéressant de choisir un silicone particulier, qui présente des caractéristiques optimales d'innocuité pour le contact cutané. Un silicone très intéressant à ce titre est le matériau commercialisé sous la marque SILBIONE®, marque déposée de la société RHODIA CHIMIE.

Pour réaliser le revêtement de la face intérieure de la tresse ou dentelle élastique, on pourra utiliser des techniques classiques en la matière, soit par des pinceaux enduits de silicone liquide, au contact desquels défile la bande à revêtir, soit en utilisant des rouleaux gravés dont les rainures sont alors remplies de silicone liquide. Après revêtement, le silicone déposé sur la bande se polymérise à l'air, la polymérisation pouvant être accélérée par le passage dans un tunnel. L'épaisseur du dépôt de silicone sera en général choisie entre 1 et 2,5 mm.

De préférence en l'espèce, il sera plus facile de réaliser d'abord la frise alvéolée, puis, dans un deuxième temps, de réaliser les deux bandes rectilignes circonférentielles qui encadrent ladite frise alvéolée. On est ainsi assuré d'avoir un bon recouvrement, avec des zones de contact bien nettes entre la frise alvéolée et les deux bandes parallèles. L'ensemble du dépôt siliconé constitue alors un tout qui procure à la fois une résistance optimale au glissement, et ce même dans le cas de membres ramollis ou présentant une pilosité excessive, et un coefficient d'aération très satisfaisant. En outre, les structures précitées permettent d'éviter tout risque de rupture du crin qui pourrait sortir du côté extérieur ou intérieur de la bande élastique, et provoquer ainsi une gêne considérable pour le porteur de l'article de contention.

L'invention n'est pas limitée au mode de réalisation qui vient d'être décrit, mais englobe au contraire toute variante reprenant, avec des moyens équivalents, les caractéristiques essentielles énoncées plus haut.

## Revendications

1. Article de contention à usage médical utilisé pour une jambe ou un bras, comportant une partie principale (11 ; 16) en tricot élastique servant à comprimer le membre sur lequel l'article est enfilé, laquelle partie principale se prolonge supérieurement par une bande élastique de maintien (20) formée d'une tresse ou d'une dentelle élastique (21) revêtue, du côté intérieur, d'un motif (23) réalisé en un matériau d'anti-glissement, **caractérisé en ce que** le motif d'anti-glissement (23) est constitué par deux bandes rectilignes circonférentielles (24, 25) essentiellement parallèles, agencées à distance l'une de l'autre, et connectées entre elles par une frise alvéolée (26) .

2. Article de contention selon la revendication 1, **caractérisé en ce que** la frise alvéolée (26) a une surface totale qui est notablement supérieure à celle de la partie associée (27, 28) de la tresse ou dentelle élastique (21) non revêtue.

3. Article de contention selon la revendication 2, **caractérisé en ce que** la surface totale de la frise alvéolée (26) représente 60 à 70% de la surface délimitée par les deux bandes rectilignes parallèles (24, 25).

4. Article de contention selon l'une des revendications 1 à 3, **caractérisé en ce que** la frise alvéolée (26) est agencée selon un tracé périodique tangentant chacune des bandes rectilignes parallèles (24, 25) .

5. Article de contention selon la revendication 4, **caractérisé en ce que** le tracé périodique comporte au moins une bande (26.1, 26.2) d'allure sinusoïdale ou en zig-zag.

6. Article de contention selon la revendication 5, **caractérisé en ce que** le tracé périodique comporte deux bandes sinusoïdales ou en zig-zag (26.1, 26.2) superposées en opposition de phase, lesdites bandes sinusoïdales étant en contact mutuel par leurs sommets adjacents, les autres sommets étant quant à eux au contact des bandes rectilignes parallèles (24, 25).

7. Article de contention selon la revendication 6, **caractérisé en ce que** les zones de contact entre bandes adjacentes ont une longueur circonférentielle (d3) au moins égale à la largeur (d1) des deux bandes rectilignes parallèles (24, 25).

8. Article de contention selon l'une des revendications 5 à 7, **caractérisé en ce que** la ou chaque bande sinusoïdale ou en zig-zag (26.1, 26.2) a une largeur (d2) sensiblement égale à la largeur (d1) des deux bandes rectilignes parallèles (24, 25).
